# EUROPÄISCHE PATENTANMELDUNG

(11) **EP 0 715 165 A1**
(43) Veröffentlichungstag der Anmeldung: **05.06.1996**
(21) Anmeldenummer: 95117847.4
(22) Anmeldetag: 13.11.1995
(51) Int. Cl.: G01N 21/89, G01N 33/36

(54) **Verfahren und Vorrichtung zur Erfassung von Eigenschaften an langgestreckten Körpern**

(30) Priorität: 29.11.1994 CH 3599/94
(71) Anmelder: ZELLWEGER LUWA AG, CH-8610 Uster (CH)
(72) Erfinder: Feller, Peter, CH-8121 Benglen (CH); Wampfler, Hans, Dr., CH-8049 Zürich (CH)
(74) Vertreter: Ellenberger, Maurice

(57) **Zusammenfassung**

Die Erfindung betrifft ein Verfahren und eine Vorrichtung bei denen ein Körper (1), ein Vorgarn oder ein Band mit einem einfachen, kostengünstigen Sensor (5) kontinuierlich gemessen wird. Ein weiterer aufwendigerer und genauer aber langsamer arbeitender Sensor (8) wird vom einfacheren Sensor getriggert falls jener besondere Ereignisse anzeigt. Diese Ereignisse werden dann vom aufwendigen Sensor gespeichert und anschliessend mit grösserem Zeitaufwand genau analysiert. Für diese Analyse kann die ganze Zeitspanne ausgenützt werden, die zwischen zwei besonderen Ereignissen liegt.

## Beschreibung

Die Erfindung betrifft eine Vorrichtung zur Erfassung charakteristischer Eigenschaften an einem langgestreckten Körper, der in Längsrichtung bewegt ist und mit Hilfe eines Sensors erfasst wird.

Als langgestreckte Körper im Sinne der Erfindung sind beispielsweise textile Garne, Seile, Drähte, Bänder usw. zu verstehen.

Solche Vorrichtungen sind bereits bestens bekannt und ermöglichen es, mit geeigneten Sensoren, die verschiedenartigsten Eigenschaften beispielsweise an einem kontinuierlich bewegten Garn zu messen. Da immer höhere Leistungen angestrebt werden, geht die Tendenz dahin, das Garn schneller herzustellen oder zu prüfen. Damit soll das Garn auch nachfolgende Verarbeitungsstufen oder Prüfungen im Labor schneller durchlaufen. Bei hohen Geschwindigkeiten wird es für herkömmliche Sensoren immer schwieriger auch Unregelmässigkeiten kleiner Ausdehnung am laufenden Garn zuverlässig zu erfassen. Damit leidet die Qualität der Erfassung der einzelnen Unregelmässigkeiten.

Zur Hebung der Qualität der Überwachung ist es denkbar, Sensoren einzusetzen, die umfassendere Informationen über Unregelmässigkeiten liefern können. Ein Beispiel für einen solchen Sensor ist eine Fernsehkamera mit nachgeschalteter Bildverarbeitung, die ja nicht nur die Unregelmässigkeit an sich erkennbar macht, sondern auch noch Angaben über die Beschaffenheit der Unregelmässigkeit abgeben kann. Für ein schnellaufendes Garn, das mit einer Geschwindigkeit von 400m/min bewegt wird und das mit einer Fernsehkamera erfasst wird, die mit einer Taktfrequenz von 50Hz arbeitet, bedeutet dies aber, dass die erfassten Halbbilder Garnabschnitte zeigen, die um 130mm auseinanderliegen. Rechnet man mit einer Halbbild-Auflösung von 160 Linien, so ergibt das auf dem Garn eine Auflösungsgrenze von 0.8mm. Dies ist für eine genaue Analyse des Garnes ungenügend. Setzt man eine Spezialkamera mit höherer Bildfrequenz ein, so verbessert man damit die Auflösungsgrenze, steigert damit aber auch den Rechenaufwand bei der Bildverarbeitung erheblich. Die dazu notwendige und genügend leistungsfähige spezielle Hardware muss auf das unmittelbar vorliegende Problem, das es zu beobachten gilt, ausgerichtet sein, was auch bedeutet, dass diese Hardware unflexibel ist. Diese Schwierigkeit könnte dadurch umgangen werden, dass man keine lückenlose Überwachung des Garnes mehr anstrebt. Dann hat man zwar eine sehr gute Erfassung der Garneigenschaften auf bestimmten Abschnitten, dagegen überhaupt keine Überwachung mehr auf anderen Abschnitten.

Die Erfindung, wie sie in den Patentansprüchen gekennzeichnet ist, löst die Aufgabe, ein Verfahren und eine Vorrichtung zu schaffen, mit der ein schnellaufendes Garn lückenlos und mit sehr kleiner Auflösung im Hinblick auf gewisse Eigenschaften überwacht werden kann.

Erfindungsgemäss wird dies durch den Einsatz von zwei verschiedenartigen und miteinander verbundenen Sensoren erreicht, wobei ein erster Sensor das Garn laufend auf gewisse Eigenschaften hin überwacht und beim Eintreffen bestimmter Ereignisse den zweiten Sensor aktiviert, der während einer begrenzten Zeit das Garn auf weitere Eigenschaften genauer untersucht. Eine solche Vorrichtung könnte beispielsweise einen optisch oder kapazitiv arbeitenden ersten Sensor und einen als Fernsehkamera ausgebildeten zweiten Sensor umfassen. Doch sind andere Kombinationen von Zwei Sensoren, die nach unterschiedlichen Messprinzipien arbeiten ebenfalls denkbar, denn eine solche Kombination kann ja ausdrücklich auf die zu untersuchenden Eigenschaften Rücksicht nehmen und so ausgebildet sein, dass die gesuchten Eigenschaften optimal erfasst werden.

Die durch die Vorrichtung erreichten Vorteile sind insbesondere darin zu sehen, dass einerseits die zu erfassenden Eigenschaften optimal erfasst und mit beliebiger Genauigkeit dargestellt werden können und dass andererseits die Auswertung der anfallenden Daten vereinfacht wird, indem dafür gesorgt ist, dass die Menge der anfallenden Daten beschränkt wird. Damit können die Resultate auch schnell ausgegeben werden und stehen damit praktisch unmittelbar nach der Erfassung zur Verfügung. Durch eine geschickte und genau problembezogene Auswahl und Kombination der Sensoren kann weiter für eine optimale Erfassung beliebiger Eigenschaften oder Daten von einem schnellaufenden Garn gesorgt werden.

Im folgenden wird die Erfindung anhand von Beispielen und mit Bezug auf die beiliegenden Zeichnungen näher erläutert, wobei
Figur 1 eine schematische Darstellung einer ersten Ausführung,
Figur 2 eine entsprechende Darstellung einer zweiten Ausführung,
Figur 3 eine entsprechende Darstellung einer dritten Ausführung,
Figur 4 eine entsprechende Darstellung einer vierten Ausführung
Figur 5 eine entsprechende Darstellung einer fünften Ausführung,
Figur 6 eine entsprechende Darstellung einer sechsten Ausführung und
Figur 7 eine entsprechende Darstellung einer siebten Ausführung der erfindungsgemässen Vorrichtung zeigen.

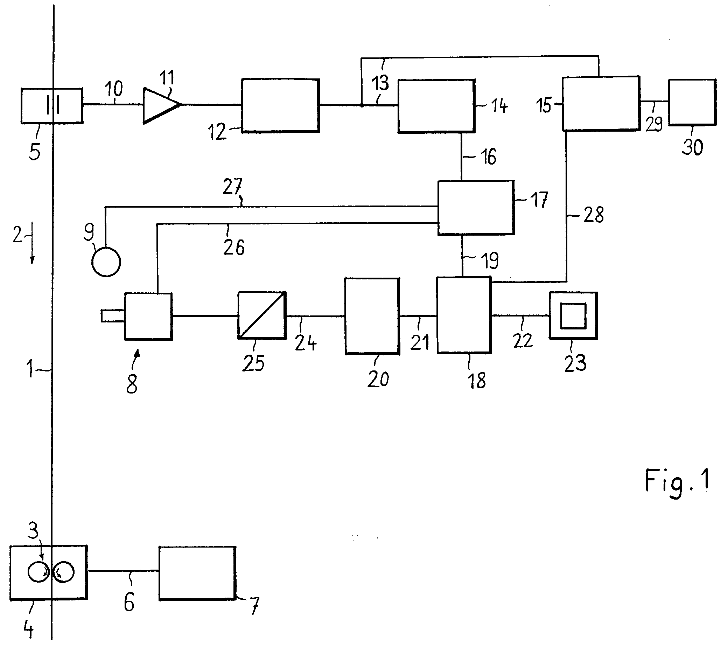

Figur 1 zeigt ein erstes Beispiel einer erfindungsgemässen Vorrichtung. Darin erkennt man einen langgestreckten Körper 1, der beispielsweise als Garn, Vorgarn, Band, optische Faser, Seil, Kabel, Rohr usw. ausgebildet sein kann, der in der Richtung eines Pfeiles 2 bewegt wird indem er durch Antriebswalzen 3 einer Antriebsvorrichtung 4 gezogen wird und dabei auch durch einen Sensor 5, der hier als kapazitiv arbeitendender Sensor dargestellt ist, erfasst wird. Über eine Leitung 6 ist die Antriebsvorrichtung mit einer Motorsteuerung 7 verbunden. Ein weiterer Sensor 8, hier als Fernsehkamera dargestellt, ist im Bereiche des Körpers 1 so aufgestellt, dass er ein optimales Bild des Körpers 1 wiedergeben kann. Daneben ist wahlweise auch eine Beleuchtungseinheit 9 vorgesehen, die den Körper zu einer Zeit und in einem Bereiche beleuchtet, der im Bild wiedergegeben werden soll.

Der Sensor 5 ist über eine Leitung 10 mit einem Signalverstärker 11 und einer Signalverarbeitungseinheit 12 verbunden. Im Zusammenhang mit Garnen betrachtet, bilden diese Elemente zusammen einen an sich bekannten Garntester. Die Signalverarbeitungseinheit 12 verarbeitet die eintreffenden Signale indem sie beispielsweise die eintreffenden Werte mit vorgegebenen Werten, die es erlauben zulässige Werte von unzulässigen Werten zu unterscheiden, vergleicht und so darüber entscheiden kann, ob ein Wert zulässig ist oder nicht. Über Leitungen 13 ist die Signalverarbeitungseinheit auch an eine Triggerschaltung 14 und einen Hauptrechner 15 angeschlossen. Dieser steuert aufgrund des darin geladenen Programmes die Abläufe in den einzelenen Elementen dieser Vorrichtung, indem er diese zur richtigen Zeit anweist, die betreffenden Funktionen auszuführen. Die Triggerschaltung 14 wandelt die Signale aus der Signalverarbeitungsenheit 12 in entsprechende Steuersignale für den weiteren Sensor 8 um und ist dazu über eine Leitung 16 mit einer Steuereinheit 17 für den weiteren Sensor 8 und diese wiederum über eine Leitung 19 mit einem Rechner 18 verbunden. Dieser ist einerseits über einen Bus 21 mit Bildspeichern 20 und andererseits über eine Leitung 22 mit einem Bildschirm, als Ausgabeeinheit 23 verbunden. Die Bildspeicher 20 sind über einen Bus 24 und über einen Analog/Digital-Wandler 25 mit dem Sensor 8, also hier der Kamera verbunden. Weitere Verbindungen 26 und 27 sind zwischen der Steuereinheit 17 und dem Sensor 8 einerseits und der Beleuchtungseinheit 9 andererseits vorgesehen. Verbindungen 28 und 29 verbinden ferner den Hauptrechner 15 mit dem Rechner 18 und mit einer Datenausgabeeinheit 30, hier beispielsweise ein Drucker. Als Sensor 8 kann sowohl eine Matrixkamera wie auch eine Zeilenkamera vorgesehen sein. Vorzugsweise sind zwei Bildspeicher 20 vorgesehen, um stets einen Bildspeicher für neue Daten frei zu haben, während der andere Speicher die auszuwertenden Daten enthalten kann.

Die Vorrichtung gemäss Fig. 1 eignet sich beispielsweise dazu, ein Garn auf die Menge und die Art von Nissen zu untersuchen. Die Nissen bilden Dickstellen im Garn 1, die durch den Sensor 5 erfasst werden. Je nach der gemachten und in der Signalverarbeitungseiheit 12 gespeicherten Vorgaben, werden alle Nissen oder nur besonders grosse Nissen für eine nähere Betrachtung durch den Sensor 8 ausgesondert. Ist eine Nisse einmal durch die Signalverarbeitungseinheit 12, die auch als Teil des Sensors aufgefasst werden kann, ausgewählt, so betätigt die Triggerschaltung 14 die Steuerschaltung 17 für den Sensor 8, die bewirkt, dass nach einer Zeitverzögerung, die einem Abstand zwischen den Sensoren 5 und 8 entspricht, der Sensor 8 und die Beleuchtungseinheit 9 aktiviert werden. Die Signale, die der Sensor 8 abgibt, in diesem Falle die Bilder aus der Kamera, werden im A/D-Wandler 25 digitalisiert und anschliessend im Bildspeicher 20 abgelegt. Nun können diese Bilder entweder direkt am Bildschirm 23 durch persönliche Betrachtung ausgewertet werden, oder die Bilder können im Rechner 18 weiterverarbeitet werden, indem sie beispielsweise mit dort abgespeicherten Mustern verglichen werden und nur ein Bild der Differenzen zum Muster zur Betrachtung abgegeben wird. Oder, der Rechner misst die Ausdehnung der Nisse und gibt gleichzeitig diese Messwerte aus. Statt einer Ausgabe von genauen Ausmassen der Nisse kann der Rechner 18 die Nisse auch einer vorbestimmten Klasse zuweisen, die gleichartige Nissen zusammenfasst. Der Rechner 18 kann dann eben eine feinere Klassierung durchführen oder auch neue Kennwerte berücksichtigen. Der Rechner 18 kann auch eine Simulation eines Gewebes vornehmen, das aus dem Garn mit diesen Nissen entstehen würde, wie dies aus der Europäischen Patentanmeldung Nr. 0 578 975 bekannt ist.

In ähnlicher Weise kann mit der Vorrichtung gemäss Fig. 1 die Haarigkeit von Garn untersucht werden. Dann ist der Sensor 5 als Haarigkeitssensor ausgebildet und meldet spezielle Haarigkeitsstellen, evtl. sog. Haarigkeitsnester, also Stellen wo das Garn besonders viele Haare hat. Solche Stellen können im Sensor 8, hier ebenfalls eine Kamera, genauer untersucht werden.

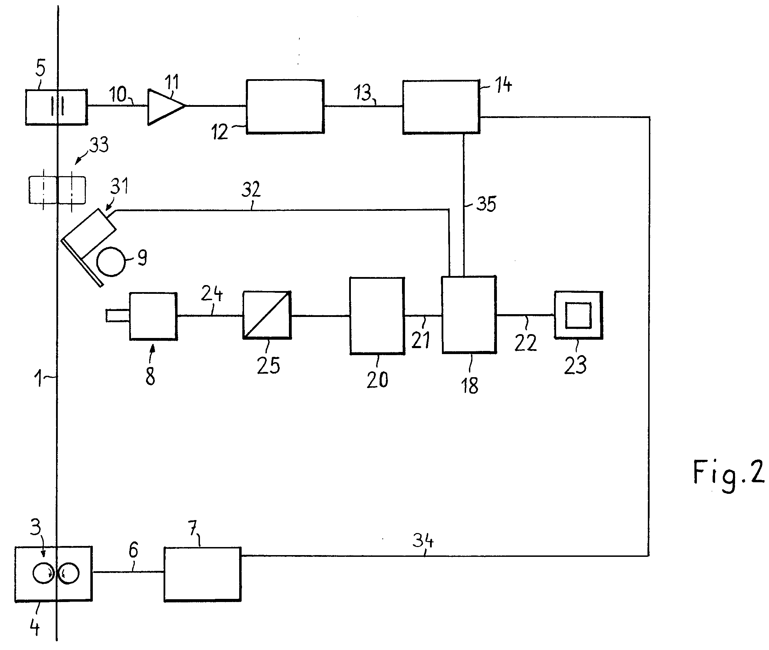

Gemäss Figur 2, in der entsprechende Elemente zu Fig. 1 mit den gleichen Bezugszeichen versehen sind, ist die erfindungsgemässe Vorrichtung zusätzlich mit einer Filtereinheit 31 ausgerüstet, die der Beleuchtungseinheit 9 vorgeschaltet ist. Die Filtereinheit 31 ist über eine Leitung 32 mit dem Rechner 18 verbunden und erlaubt es spezielle Lichtbedingungen für den Sensor 8 zu schaffen. Damit kann beispielsweise eine Nissen- oder Fremdstoffanalyse in verschiedenen Spektralbereichen durchgeführt werden, indem das geeignete Filter vorgeschaltet wird. Der Körper 1 kann auch verdreht oder in eine andere Position gedreht werden bevor er in den Bereich des Sensors 8 gelangt. Dazu ist beispielsweise ein angetriebenes Walzenpaar 33 vorgesehen. Dazu kann eventuell auch der Vorschub des Körpers 1 kurzzeitig ausgeschaltet werden, wozu die Motorsteuerung 7 über eine Leitung 34 mit der Triggerschaltung 14 verbunden ist. Als Variante ist hier die Triggerschaltung über eine Leitung 35 direkt am Rechner 18 angeschlossen, der auch über die Leitung 32 die Filtereinheit 31 steuert.

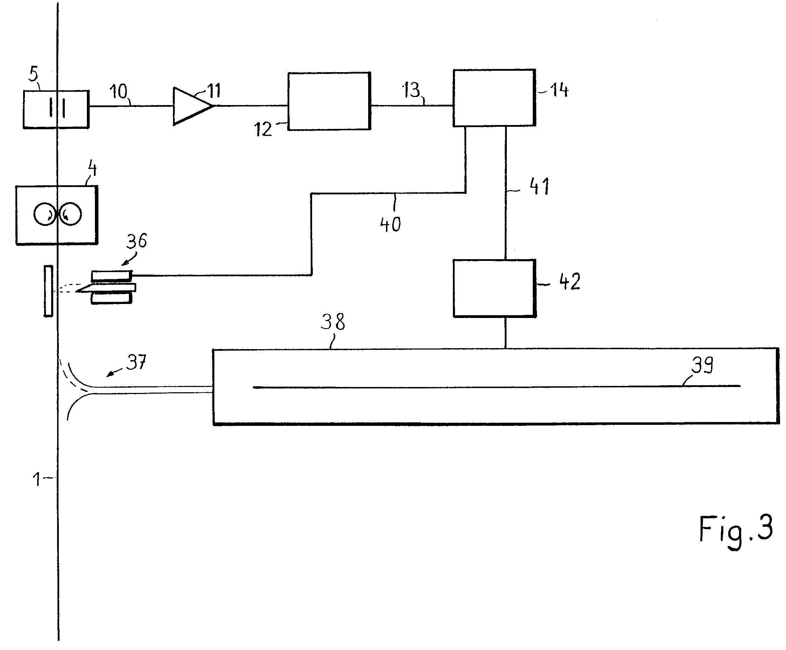

Figur 3 zeigt wiederum die bereits bekannte Serieschaltung der Elemente 5, 10, 11, 12, 13 und 14 sowie die Antriebsvorrichtung 4, der nachgeschaltet nun ein Messer 36 angeordnet ist. Mit dieser Vorrichtung können Muster 39 entnommen werden, die mit dem Messer 36 abgetrennt werden und die hier beispielsweise über eine Saugvorrichtung 37 in eine Prüfeinrichtung 38 geleitet werden. Solche Messer 36 sind bereits aus handelsüblichen Garnreinigern bekannt. Ebenso bekannt sind Saugvorrichtungen 37, die bei Textilmaschinen oder Laborgeräten anzutreffen sind. Leitungen 40 und 41 verbinden die Triggerschaltung 14 einerseits mit dem Messer 36 und andererseits mit einer Steuerung 42 für die Prüfeinrichtung 38. Diese Prüfeinrichtung 38 hat die Funktion eines weiteren Sensors, der genauere Untersuchungen der Eigenschaften des Körpers zulässt. Dieser Sensor kann z.B. als Reissprüfer ausgebildet sein. Damit kann man beispielsweise im Sensor 5 eine Dünnstelle ermitteln, dann eine Probe mit dieser Dünnstelle abzweigen und diese Probe auf ihre Festigkeit prüfen. Damit kennt man die Reissfestigkeit dieser Dünnstelle und kann gleichartige Dünnstellen, die später auftreten, dementsprechend behandeln oder bewerten, d.h. im Körper 1 belassen oder systematisch herausschneiden. Ähnliche Prüfungen lassen sich neben Garnen auch für Körper anderer Art in diesem Sinne durchführen.

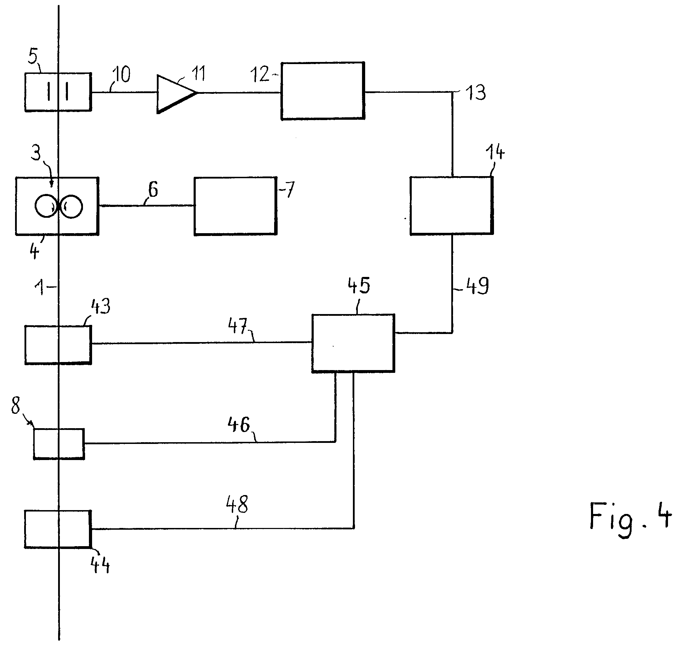

Die Figur 4 zeigt neben den bereits bekannten Elementen, die mit dem Sensor 5 und dem Antrieb 4 zusammenarbeiten, einen weiteren Sensor 8, Aktoren 43 und 44 sowie eine Auswerteschaltung 45 die mit diesen über Leitungen 46, 47 und 48 verbunden ist. Eine Leitung 49 verbindet die Auswerteschaltung 45 mit der Triggerschaltung 14. Mit den Aktoren 43 und 44 kann der Körper 1 in einer bestimmten Weise beeinflusst werden. Dabei misst der entsprechend geeignet ausgebildete Sensor 8 die erzielte Wirkung. Beispielsweise können die Aktoren 43 und 44 den Körper unter Zugspannung setzen und der Sensor 8 misst diese Spannung. Oder, die Aktoren 43 und 44 verdrehen den Körper 1 und der Sensor 8 misst diese Verdrehung oder andere Grössen, die sich mit der Verdrehung ändern.

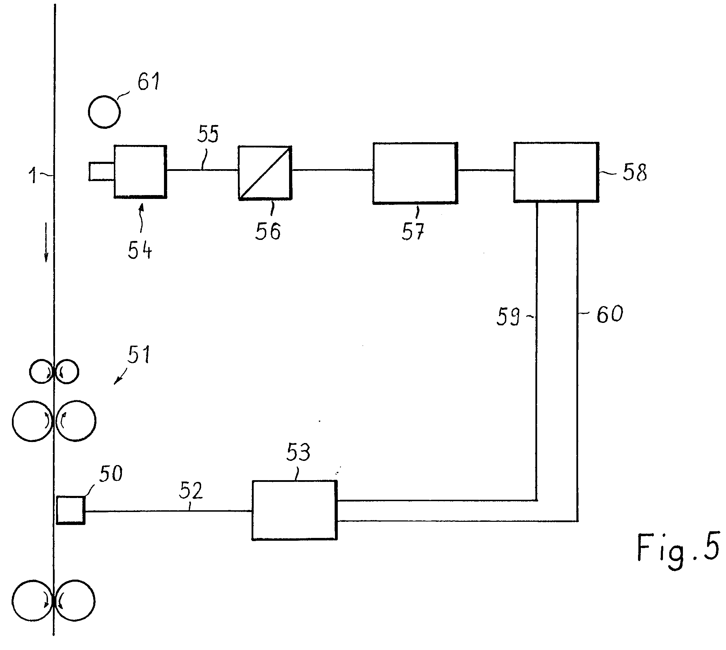

Eine weitere Ausführung ist in Figur 5 gezeigt. Dabei ist ein erster Sensor 50 beispielsweise in ein kontinuierlich arbeitendes Garnzugprüfgerät 51 integriert, das die Reissfestigkeit des Körpers 1, hier eines Garnes oder einer Faser, in bekannter Weise prüfen kann. Der Sensor 50 ist über eine Leitung 52 an eine Rechnereinheit 53 angeschlossen. Als weiterer Sensor 54 ist hier ebenfalls eine Zeilenkamera vorgesehen, die über eine Leitung 55, einen A/D-Wandler 56 und einen Ringspeicher 57 an eine Verarbeitungseinheit 58 angeschlossen ist. Leitungen 59 und 60 verbinden diese mit der Rechnereinheit 53. In nun bereits bekannter Weise ist auch eine Beleuchtungseinheit 61 vorgesehen. Bei dieser Vorrichtung wird nun der Körper 1 zuerst durch den weiteren Sensor 54 laufend beobachtet, in diesem Falle in einer Bildfolge erfasst. Diese Bildfolge wird während einer vorgegebenen Zeit im Ringspeicher 57 gespeichert und dann, wenn der Ringspeicher gefüllt ist, durch neuere Bilder überschrieben. Wird im nachgeschalteten Sensor 50 beispielsweise ein besonders tiefer Wert für die Reissfestigkeit ermittelt, so kann die Rechnereinheit 53 über die Leitungen 59 und 60 die Verarbeitungseinheit 58 anweisen, die Bilder dieser Garnprobe aus dem Ringspeicher 57 zu entnehmen, oder das Überschreiben im Ringspeicher 57 zu unterbrechen. So kann diese Garnprobe untersucht werden und vielleicht ein Hinweis auf die Ursache des Bruches gefunden werden.

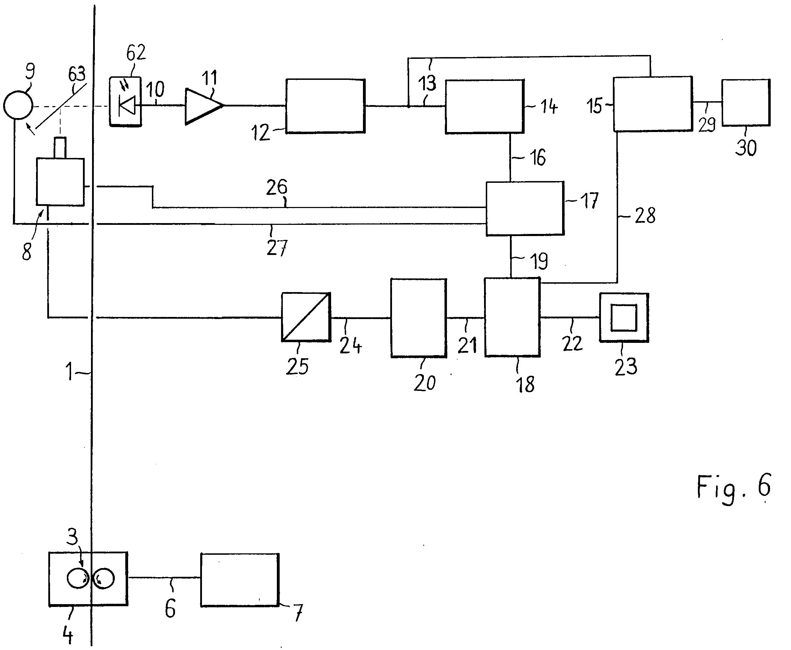

Eine weitere Ausführung ist in Figur 6 gezeigt. Diese weist für den Körper 1 einen optischen Sensor 62 auf, hier als Photodiode ausgebildet, der mit einer Lichtquelle 9 und einem weiteren Sensor 8 zusammenarbeitet, wie sie bereits aus der Fig. 1 bekannt sind. Der Unterschied zur Ausführung gemäss Fig. 1 besteht hier darin, dass einerseits die Lichtquelle 9 mit einem optischen Sensor 62 und andererseits mit einer Kamera als weiteren Sensor zusammenarbeitet. Ein Spiegel 63, der als halbdurchlässiger Spiegel ausgebildet sein kann, oder der dann weggeklappt wird, wenn der weitere Sensor 8 aktiviert ist, ermöglicht es, dass der weitere Sensor 8 den Körper zur gleichen Zeit und am gleichen Ort erfassen kann wie der erste Sensor 62. Die weiteren Elemente sind bereits aus der Fig. 1 bekannt und auch gleich bezeichnet und deshalb hier nicht weiter beschrieben.

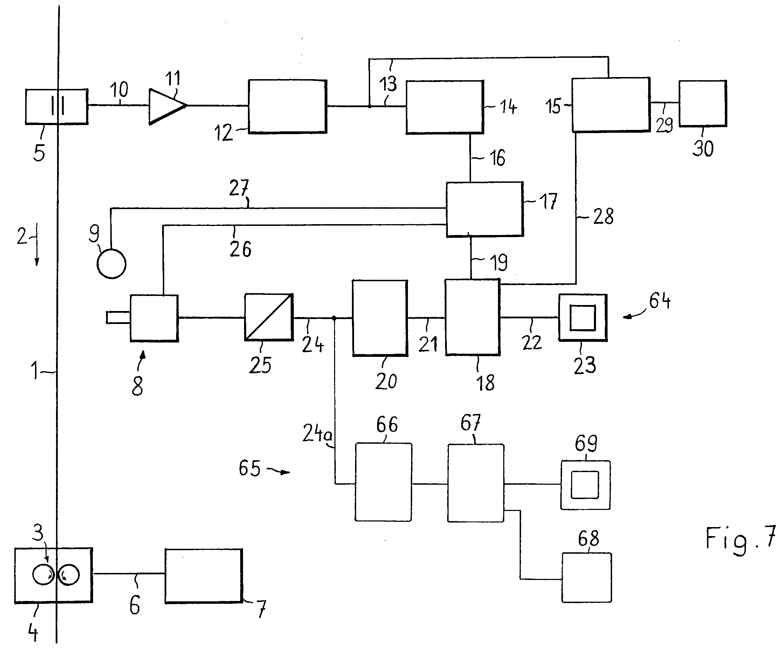

Eine weitere und von der in Figur 1 gezeigten Ausführung abweichende Variante ist in der Figur 7 dargestellt. Für gleiche Elemente sind deshalb auch die gleichen Bezugszeichen verwendet. Dabei sind für den Sensor 8 zwei verschieden ausgebildete Pfade 64 und 65 für die Verarbeitung der Signale vorgesehen. Der Pfad 64 weist die aus der Fig. 1 bekannten Elemente auf während der Pfad 65 einen Ringspeicher 66, einen Bildverarbeitungsprozessor 67 und, daran angeschlossen, einen Drucker 68 und oder einen Bildschirm 69 aufweist. Der Ringspeicher 66 ist dabei über eine Leitung 24a mit der Leitung 24 verbunden und mit dem Bildspeicher 20 parallelgeschaltet. Der Bildverarbeitungsprozessor 67 ist sowohl an den Ringspeicher 66 als auch an den Rechner 18 angeschlossen. Mit dieser Ausführung kann einerseits eine genauere Untersuchung des Körpers 1 an zeitlich vorausbestimmten Stellen wie auch eine Untersuchung von Stellen am Köper erfolgen, die zeitlich nachlaufend angegeben wurden. Wenn also eine interssante Stelle erkannt ist, kann sie einerseits unmittelbar anschliessend genauer untersucht werden und kann andererseits auch nach dieser genaueren Untersuchung noch vertieft werden, wenn die genauere Untersuchung weitere Untersuchungen nahelegt.

Mit den Vorrichtungen gemäss den Figuren 1 bis 7 kann ein Körper mit einem einfachen, kostengünstigen Sensor kontinuierlich gemessen werden. Der weitere aufwendigere und genauer aber langsamer arbeitende Sensor, oder auch seine signalverarbeitenden Elemente, werden vom einfacheren Sensor getriggert falls jener besondere Ereignisse misst. Diese Ereignisse werden dann vom aufwendigen Sensor aufgenommen und gespeichert und können anschliessend mit grösserem Zeitaufwand genau analysiert werden. Für diese Analyse kann die ganze Zeitspanne ausgenützt werden, die zwischen zwei besonderen Ereignissen liegt. Beide Sensoren müssen synchronisiert werden, damit beide Sensoren die gleiche Stelle im Körper erfassen. Eine solche erfindungsgemässe Vorrichtung lässt sich insbesondere auch auf andere textile Gebilde wie Bänder, Vliese usw. anpassen. Diese Anpassung geschieht durch die Wahl geeigneter Sensoren und Antriebsmittel. Die an die Sensoren angeschlossenen weiteren Elemente, die zur Synchronisation des weiteren Sensors und zur Verarbeitung der Signale von den Sensoren vorgesehen sind, sind allesamt an sich bekannte Elemente. Das gleiche gilt für die Programme, die für die Auswertung der Signale aus dem genauer arbeitenden Sensor benützt werden.

In der Textilindustrie sind für die Spinnerei heute Klassierungssysteme bekannt, die Kriterien vorgeben, nach denen Unregelmässigkeiten in einem Garn klassiert werden können. Der einfache kontinuierlich arbeitende Sensor kann mit solchen Kriterien arbeiten. Mit dem weiteren Sensor können für jede Klasse typische Unregelmässigkeiten in Bildern sichtbar gemacht werden, wenn sie einmal auftreten. Damit kann sich der Anwender ein viel genaueres Bild machen, welcher Art die Unregelmässigkeiten sein können, die einer bestimmten Klasse zugerechnet werden. Er kann aber auch weitere Besonderheiten an einem Garn erkennen, wie z.B. Faserschlaufen, abstehende Enden, die Länge der Fasern, die Orientierung der Fasern an der Oberfläche, die Haarigkeit im Nahbereich und im Fernbereich usw. Eine weitere Möglichkeit ergibt sich dabei den Variationskoeffizienten CV auch in sehr kurzen Bereichen z.B. zwischen 0 und 8mm genauer zu berechnen und zwar mit Hilfe der Bildbetrachtung. Mit dieser Vorrichtung lassen sich auch Schmutzteile oder Fremdkörper im Messgut erkennen und genauer analysieren. Beide Sensoren lassen sich auch so kombinieren, dass sie einander ergänzende Informationen liefern. Z.B. kann der erste Sensor kapazitiv arbeiten und so die Masse des Körpers bestimmen. Der zweite Sensor kann durch eine Bildbetrachtung den Durchmesser des Körpers anzeigen. Durch Vergleich der Angabe über die Masse und den Durchmesser kann eine zusätzliche Grösse, hier die Dichte des Körpers, bestimmt werden. Weitere hier nicht aufgeführte Anwendungen sind denkbar. Z.B. kann der einfachere kontinuierlich arbeitende Sensor auf einer Produktionsmaschine wie eine Kabelmaschine, einen Extruder für Kunststoffrohre, einer Spinnmaschine, eine Karde usw. angeordnet sein, der weitere genauer arbeitende Sensor kann dann abseits des Produktionsstranges angeordnet sein, so dass das produzierte Gut von Fall zu Fall dorthin abgezweigt wird für die Durchführung der genaueren Messung.

## Patentansprüche

1. Vorrichtung zur Erfassung charakteristischer Eigenschaften an einem langgestreckten Körper (1) der in Längsrichtung bewegt ist und mit Hilfe eines Sensors (5) erfasst wird, dadurch gekennzeichnet, dass der Sensor mit einem weiteren Sensor (8) verbunden ist, der zur genaueren Erfassung von Eigenschaften ausgebildet und angeordnet ist und zeitweise, durch den einen Sensor ausgelöst, die genauer erfassten Eigenschaften freigibt.

2. Vorrichtung nach Anspruch 1, dadurch gekennzeichnet, dass der eine Sensor (5) kontinuierlich und nach einem ersten Messprinzip arbeitet, während der weitere Sensor (8) diskontinuierlich nach einem anderen Messprinzip arbeitet.

3. Vorrichtung nach Anspruch 1, dadurch gekennzeichnet, dass der weitere Sensor zur Aufnahme und Verarbeitung von Bildern ausgebildet und angeordnet ist.

4. Vorrichtung nach Anspruch 1, dadurch gekennzeichnet, dass der weitere Sensor in Bewegungsrichtung (2) des Körpers dem einen Sensor nachgeschaltet ist.

5. Vorrichtung nach Anspruch 1, dadurch gekennzeichnet, dass Speicher (20, 57) an den weiteren Sensor (8, 54) angeschlossen sind, zur Speicherung der durch diesen erfassten Signale.

6. Vorrichtung nach Anspruch 1, dadurch gekennzeichnet, dass der weitere Sensor in Bewegungsrichtung des Körpers dem ersten Sensor vorgeschaltet ist und mit einem Ringspeicher (57), zur zeitlich beschränkten Speicherung der gemessenen Eigenschaften, verbunden ist.

7. Vorrichtung nach Anspruch 1, dadurch gekennzeichnet, dass der weitere Sensor Elemente zur weiteren Aufbereitung seiner Signale aufweist.

8. Verfahren zur Erfassung charakteristischer Eigenschaften an einem langgestreckten Körper, der in Längsrichtung bewegt wird, dadurch gekennzeichnet, dass Eigenschaften des Körpers laufend gemäss einem ersten Messprinzip und zusätzlich gemäss einem weiteren Messprinzip erfasst werden, wobei die Auswertung der gemäss einem Messprinzip erfassten Eigenschaften diskontinuierlich erfolgt.

9. Verfahren nach Anspruch 7, dadurch gekennzeichnet, dass die Eigenschaften gemäss dem einen Messprinzip mit grösserer Genauigkeit erfasst werden.
